# EUROPEAN PATENT APPLICATION

(11) **EP 2 886 124 A1**
(43) Date of publication of application: **24.06.2015**
(21) Application number: 13769895.7
(22) Date of filing: 14.03.2013
(51) Int. Cl.: A61K 36/42, A61P 35/00

(54) **RAW EXTRACT OF A SECHIUM EDULE HYBRID, METHOD FOR EXTRACTING SAME AND USE THEREOF FOR FORMULATIONS HAVING AN ANTINEOPLASTIC EFFECT**

(30) Priority: 28.03.2012 MX 2012003818
(71) Applicant: Aguiñiga Sánchez, Itzen, 09630 México D.F. (MX); Cadena Iñiguez, Jorge, 56108 Texcoco (MX); Santiago Osorio, Edelmiro, 57000 Nezahualcóyotl (MX); Cisneros Solano, Victor Manuel, 91100 Veracruz (MX); Soto Hernández, Ramón Marcos, 55710 Coacalco (MX); Arévalo Galarza, Mª. De Lourdes Catalina, 56121 Col. San Jacinto (MX); Rivera Martínez, Ana Rocío, 09090 México D.F. (MX)
(72) Inventor: Aguiñiga Sánchez, Itzen, 09630 México D.F. (MX); Cadena Iñiguez, Jorge, 56108 Texcoco (MX); Santiago Osorio, Edelmiro, 57000 Nezahualcóyotl (MX); Cisneros Solano, Victor Manuel, 91100 Veracruz (MX); Soto Hernández, Ramón Marcos, 55710 Coacalco (MX); Arévalo Galarza, Mª. De Lourdes Catalina, 56121 Col. San Jacinto (MX); Rivera Martínez, Ana Rocío, 09090 México D.F. (MX)
(74) Representative: Stiebe, Lars Magnus
(86) International application number: PCT/MX2013/000031
(87) International publication number: WO 2013/147578

(57) **Abstract**

The present invention relates to a pharmaceutical composition containing a crude extract of the hybrid H-387-07-GISeM, which is a product of which is the product of crossbred of varieties: *Sechium edule* var. *virens levis* with *Sechium edule* var. *amarus silvestrys* and the crossbred of its result with *Sechium edule* var. *nigrum spinosum,* which has therapeutic effectiveness as antineoplastic agent; said composition has an improved effect compared with its parents and also its administration has no side effects.

## Description

### Field of the invention

The present invention relates to the use of plant extracts in the formulation of compositions that have antineoplastic effect. Particularly, the present invention relates to the use of a crude extract of a hybrid of *Sechium edule* named "H-387-07-GISeM", which is useful for the treatment of cancer tumours due to its antineoplastic effect.

### Background of the invention

Cancer is a growing public health issue worldwide, affecting people of all ages, and is produced by various changes in the behaviour of cells, originated by changes in the genetic information, which cause the uncontrolled proliferation of these cells, forming malignant tumours that tend to grow invasively, destroying normal tissues and organs (Karp, 1998). Such changes in the cell are caused by accumulation of gene modifications induced by physical, chemical or biological agents (Yocota, 2000), such as ultraviolet radiation, radioactive emissions, recurrent infections with pathogenic organisms, exposure to carcinogenic chemical compounds (Weinberg 1996), damage to the immune system, endocrine imbalance, age, and physiological conditions.

Cancer is classified according to the type of tissue where it progresses, such as sarcomas from connective tissue, muscular tissue, and adipose tissue; carcinomas originated from epithelial tissue, and finally leukemia and lymphomas whose origin is the hematopoietic and lymphoid tissue.

Cancer treatment varies according to its type and status, and medicine applies different procedures to attempt to cure it. Surgery is one of these, and involves the extraction of the tumor by means of a surgery, which depends on the localization and size of the tumor and has side effects such as pain and fatigue depending on the type of surgery (NCI, 2008). Other such as radiotherapy, which relate to the application of high energy radiation to kill cancer cells in a specific area are applied externally towards the tumor area or internally by means of needles or catheters containing radioactive substances. Side effects are fatigue, depression of immune system, hair loss, as well as redness, skin dryness and sensitivity (NCI, 2008). Chemotherapy is other of the methods: drugs are used to kill the cancer cells throughout the body; however, healthy cells are damaged too, having side effects as immunosuppresion, loss of appetite, hair loss, fatigue, nausea, vomiting diarrhea and mouth sores (NCI, 2008).

All treatments generally aim to kill cancer cells leading them to initiate some process of cell death, but they also affect healthy cells, causing ultimately and as a whole the side effects.

Currently the need to strengthen the research on alternative or supplementary treatments has increased, and these have the purpose of kill tumor cells and mitigate side effects with higher specificity and efficacy in cancer treatment. In this regard, natural products are used by humans for obtaining medicinal agents (Setzer et al., 2003), using plants as source for such compounds.

Within the group of anticancer drugs derived from plants currently in clinical use, there are six main classes of compounds (including their synthetic and semi-synthetic derivatives): vinca alcaloids, epipodophyllotoxins, taxanes, camptothecines, homoharringtonines and ellipticines (Balunas et al., 2005, Vega et al., 2006), many of which are found in plants used in ethnomedicine; however, chemical composition or biological activity of about 58% of plant species used have not been studied (Vega et al., 2006).

Sechium spp, from *Cucurbitaceae* family, is commonly called "chayote". Among neotropical species of this family present in Mexico, *Sechium edule* (Jacq.) Sw. stands out and has great importance to humans due to its use in food and traditional medicine, since fruits and seeds are rich in various amino acids, and also contain seven out of nine essential amino acids (lisine, leucine, methionine, phenylalanine, valine, isoleucine, hystidine) (Flores, 1989).

Other studies with fruits, leaves and stems of chayote have revealed its diuretic (Jensen et al., 1986), anti-inflammatory (Salama et al., 1986) and hypotensive activity (Cambar et al., 1980; Guppy et al., 2000), antimutagenic (Yen et al., 2001), antibacterial (Ordonez et al., 2003), helps to remove kidney stones, is used as a complement in the treatment of arteriosclerosis and hypertension (Abdelnour et al., 2002), alleviates inflammation of skin and intestines and also favors ulcer healing (Dire et al., 2003)

It has been shown that *S. edule* (Jacq.) Sw. contains peroxidasecucurbitins, sterols, alkaloids, saponins, phenols, polyphenols, flavonoids, and cucurbitacins (Cadena-Iñiguez, 2005), with antiallergenic, anti-inflammatory, antioxidant, antiviral and antitumor effects attributed to the molecules (Salama et al., 1986; Jayaprakasam et al., 2003; Setzer et al., 2003; Siciliano et al., 2004 and Ordoñez et al., 2006), and also Wu et al. (1998), characterized the sechiumine protein of the seed, which has properties of ribosomal inactivation and could be a chemotherapeutic agent; however, the extraction process is highly complex and is limited to the extraction of the protein from the seed, so that the quantity of biological material used is very high.

It has been reported that crude extracts of eight varieties of *S*. *edule* have antiproliferative potential, where has been registered that alcohol extracts of the fruits of varieties var. *nigrum xalapensis* and var. *albus levis* have cytotoxic activity in the proliferation of L929 and P388 cell lines, and the extract of var. albus minor and var. *albus dulcis* does not inhibit proliferation (Cadena- Iñiguez et al., 2007; Salas et al., 2007), while in the HeLa cell line var. *albus levis* and *virens levis* have greater antiproliferative potential (Rivera, 2010). Sosa (2008), evaluated the aqueous, methanolic and ethanolic extracts of *S. edule* (Jacq.) Sw. var. *nigrum spinosum* in HeLa and L929 lines, where concludes that methanolic and ethanolic extracts are those that have antiproliferative and cytotoxic activity in a dose-dependent way on the L929 cell line. Subsequently, Monroy-Vázquez (2009), confirms such activity and also mentions having obtained four fractions of the extract, of which three showed similar antiproliferative activity; being 5-7 the fraction with the most activity and specificity on HeLa, indicating that chemical components of the fraction corresponded to the group of saturated fatty acid esters; expanding the range of metabolites and sources of plant species with antineoplastic activity.

A review of important medicinal plants of *Cucurbitaceae* family made by Dhiman et al. (2012) describes the cardiovascular, hepatoprotecive, anti-inflammatory and anticancer effects of the *Cucurbitaceae* family; in particular the anticancer activity of *Curcubita andrean* species and antioxidant, antihipertensive and anti-inflammatory activity of *S. edule;* however, its antineoplastic activity is not mentioned.

Patent CN1603340 discloses the use of a chayote protein with antitumor activity, in which the effect of the protein regarding the percentage of tumor inhibition is shown.

### Summary of the invention

Therefore, an object of the present invention is to provide an extract of a hybrid of *Sechium edule* named "H-387-07-GISeM" with higher antineoplastic therapeutic effectiveness than extracts from other species of the genus *Sechium.*

Another object of the present invention is to provide an extract of a hybrid of *Sechium edule* named "H-387-07-GISeM" with higher antineoplastic therapeutic effectiveness than the exerted by other drugs that inhibit the growth of tumor cells such as citarabine (Ara-C).

A further object of the present invention is to provide a method for obtaining a crude extract of a hybrid of *Sechium edule* named "H-387-07-GISeM" with high antineoplastic therapeutic effectiveness.

Also, another object of the invention is to provide an extract of a hybrid of *Sechium edule* named "H-387-07-GISeM", which produces an antineoplastic therapeutic effect and has no side effects.

These and other objects will be achieved through the invention disclosed in detail below.

### Brief description of Figures of the invention

Figure 1 depicts the dose-response curve for the effect of the extract of hybrid H-387-07-GISeM on the proliferation of normal bone marrow cells and tumor lines L929, HeLa and WEHI-3 evaluated by the crystal violet staining technique.
Figure 2 depicts the dose-response curve for the effect of the extract of hybrid H-387-07-GISeM on the proliferation of tumor cell line K-562 evaluated by the crystal violet staining technique.
Figure 3 refers to an agarose gel where the DNA fragmentation of cell line WEHI-3 treated with IC50 of hybrid H-387-07-GISeM is observed, and is compared with citarabine and a carrier, a technique used to evaluate death by apoptosis.
Figure 4 refers to the survival of mice after 6 months of treatment with 6.6 mg/kg of the extract of hybrid H-387-07-GISeM every 48 hrs intraperitoneally for 25 weeks, and 12 additional weeks with no treatment.

### Detailed description of the invention

The present invention relates to a crude extract *Sechium edule* hybrid named "H-387-07-GISeM ", which is the product of crossbred of varieties: *Sechium edule* var. *virens levis* with *Sechium edule* var. *amarus silvestres* and the crossbred of its result with *Sechium edule* var. *nigrum spinosum,* which has therapeutic effectiveness as antineoplastic agent, and the method for obtaining such extract.

According to the present invention, a preferred way to obtain the extract of hybrid H-387-Q7-GISeM, is the following:
1. Cut off the fruits (including epidermis, spines and seeds) and vegetative parts of hybrid H-387-07-GISeM into small pieces.
2. Dry at 40°C with air circulation and grind the pieces to standardize the particle size.
3. Extract the dried material of hybrid H-387-07-GISeM by discontinuous extraction with an organic or aqueous solvent, recirculating the solvent at least 7 times. The organic or aqueous solvent is selected from the group comprising methanol, ethanol, hexane and water.
4. Evaporate the solvent under reduced pressure to obtain the crude extract.
5. Dilute the crude extract in ethanol in 3:1 ratio.
6. Dilute to one milliliter with PBS (Phosphate Buffer Solution) and homogenize using a vortex.
7. Centrifuge and collect the supernatant.
8. Make dilutions using only the supernatant.
9. Sterilize for 40 min with UV light.
10. Store the dilutions at 4°C.

A biochemical analysis to the extract of *Sechium edule* hybrid named "H-387-07-GISeM" was performed. The content of secondary metabolites indicates that the extract of *S. edule* named "H-387-07-GISeM" has a high content of triterpenes and phenols, a medium content of flavonoids, fatty acid esters, and saponins, and a low content of saponins; each secondary metabolite group is associated with some kind of antitumor activity (Table 1).

**Table 1. Composition of secondary metabolites of the hybrid H-387-07-GISeM.**

| **Secondary Metabolites** | |
|---|---|
| Terpene (mono, di, tri, tetra and penta) | ++++ |
| Phenols | +++ |
| Flavonoids | ++ |
| Tannins | + |
| Saponins | ++ |
| Fatty acid esters | ++ |

Where each "+" represents about 7.1% of each secondary metabolite, and all them added represent 100%.

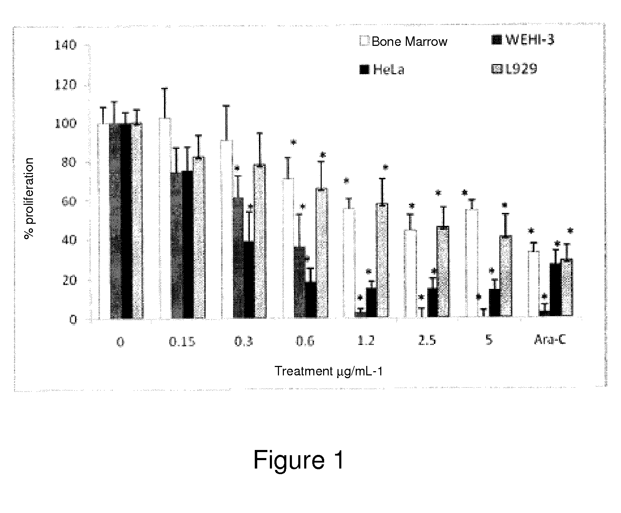

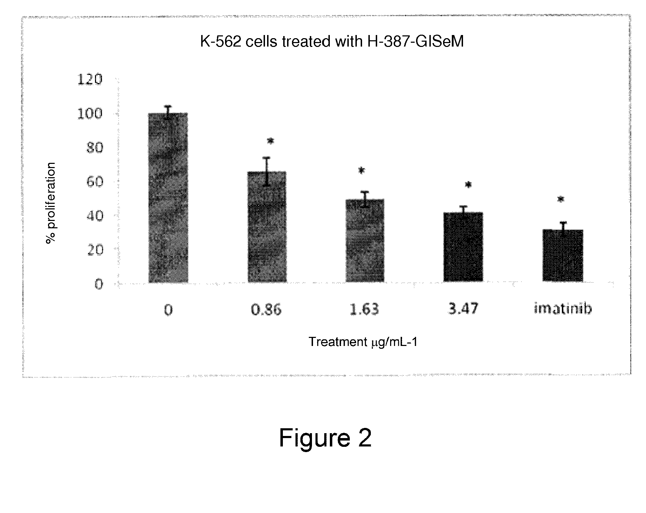

To determine the effect of the extract of *Sechium edule* hybrid named MH-387-07-GISeM" on tumor cells, HeLa cell lines, which are derived from adenocarcinoma of woman's cervical epithelial tissue; cell line L929 derived from male mouse's fibrosarcoma of areolar and adipose subcutaneous connective lung tissue, WEHI-3 y K562 are lines derived from leukemia (liquid tumors) which are maintained in IMDM culture medium (Iscove's Modified Dulbecco's Medium). Tumor cells were cultured for 72 hours in 200 µL of culture medium and serum containing 0.0, 0.15, 0.3, 0.6, 1.2, 2.5 and 5 µg/mL of extract, and were evaluated by the crystal violet technique (Figure 1). Cells were maintained in culture for 5 days after being stimulated with diverse doses of extract or Ara-C as a control (5 µM), and were evaluated by the crystal violet technique, Figure 1, where a dose-response curve of the effect of H-387-07-GISeM extract on cell proliferation of lines EHI-3, HeLa, L929, and bone marrow cells was plotted. Values are the average of three tests with tree replicates. Lines above the bars indicate ± standard deviation. * Significant difference (p <0.05) compared to control. The extract of *Sechium edule* hybrid named "H-387-07-GISeM" inhibits the proliferation in a dose-dependent way, and only was required less than 1.2 µg/L to completely inhibit the proliferation of cell lines of cervical carcinoma solid tumors (HeLa) or mouse leukemic line EHI-3, whereas for the mouse lung fibrosarcoma (L929) no dose totally blocks it. On the other hand, normal bone marrow cells of mouse treated with 5 µg/mL achieve a proliferation of 60% compared to the control, but with 5 g/mL of citarabine (Ara-c) it only reaches a 35%. The use of 5 µm/mL of *Sechium edule* hybrid named "H-387-07-GISeM" in HeLa killed all cells, whereas the antineoplastic in clinical use did not (Figure 1), so that this carcinoma is more sensitive to the extract of the invention. The extract of *Sechium edule* hybrid named "H-387-07-GISeM" also inhibits the proliferation of K562 in a dose-dependent way (Figure 2) and in a way similar to imatinib, a chemotherapeutic in clinical use for treating chronic myeloid leukemia, a pathology from which this cell line was obtained and whose success is based on the use of imatinib ant its analogues, without being curative.

Figure 2 describes a dose-response curve depicting the effect of the extract of H-387-07-GISeM on cell proliferation of K-562 line. Values are the average of three tests with tree replicates. Lines above the bars indicate ± standard deviation. * Significant difference (p <0.05) compared to control.

Also, to determine the mean inhibitory concentration (IC₅₀), i.e., the concentration of extract required to inhibit cell proliferation by 50 % (IC₅₀) of each cell line, the equation of linear regression of the dose-response of proliferation curve corresponding to cell lines treated with extract of *Sechium edule* hybrid named "H-387-07-GISeM" was used.

With the proliferation data obtained in bioassays, the concentrations required to halve the cell proliferation (IC₅₀) were determined. Results indicate that cells of cervical carcinoma (HeLa) are 17-fold more sensitive than those of normal bone marrow, followed by acute myeloid leukemia (WEHI-3) (8-fold), chronic myeloid leukemia (K562) (2.5-fold) and lung fibrosarcoma (L929) (2-fold) (Table 2).

**Table 2. IC₅₀ concentrations (ug_{˙}mL⁻¹) of H-387-07-GISeM extract on tumor and normal cell lines, calculated based on the dose-response curve by a linear regression equation.**

| **Cell line** | **IC₅₀ (µg·mL⁻¹)** |
|---|---|
| **WEHI-3** | 0.50 |
| **K-562** | 1.50 |
| **HeLa** | 0.20 |
| **L929** | 1.78 |
| **Normal bone marrow** | 3.90 |

A further way to evaluate the effect of the extract of *Sechium edule* hybrid named "H-387-07-GISeM" on tumor cells was to determine the presence of apoptotic bodies through morphology; for this purpose, 1x10⁵ cells/mL of lines HeLa, L929 and WEHI-3 were obtained and were grown for 48 hours in 1000 µL of culture medium and sera in each well of 24-well, flat-bottomed, ultra-low adherence plates (Costar, Cambridge, MA, E. U. A.), with or without the IC50 of the diverse extracts for each cell line. Once elapsed that time, cells were collected and placed on a slide; then were fixed with 1 mL of methanol and stained for 20 minutes with 10% Giemsa stain. Cells were observed under a microscope (Cari Zeiss, Primo Star, Germany) equipped with 100x objective lens. Morphological changes like decreased cell volume, chromatin condensation, loss of cell membrane regularity, were cytomorphological parameters evaluated in the formation of apoptotic bodies. 200 cells of each experiment were counted to obtain the percentage of normal and apoptotic cells. Tumor lines show apoptotic bodies (Table 3), but normal bone marrow cells do not.

**Table 3. Percentage of apoptotic bodies induced on three cell lines after 48 hrs of stimulation with the IC50 of the extract of Sechium edule hybrid named "H-387-07-GISeM" for each line, control and Ara-C 5 µg/mL, respectively. Values are presented as mean ± standard deviation.**

| | **% Apoptotic bodies** | | |
|---|---|---|---|
| **Cell line** | **0** | **H-387-GISeM** | **Ara-C** |
| **WEHI-3** | 0.5 ± 0.71 | 53 ± 9.4 | 61.5 ± 8.2 |
| **HeLa** | 0 ± 0 | 3 ± 1.4 | 21 ± 0 |
| **L929** | 0 ± 0 | 7 ± 4.2 | 6 ± 1.4 |

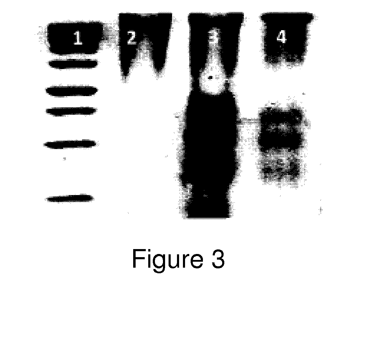

Apoptosis induction was evaluated through DNA fragmentation. Data show that WEHI-3 treated with CUCURBIN-387 experiences DNA fragmentation, as well as Ara-C does (Figure 3), where the DNA fragmentation of WEHI-3 cells grown for 48 hours with various treatments is described. 1. - Molecular weight marker; 2. - Control; 3. - H-387-07-GISeM; 4. - ARA-C.

On the other hand, the cell viability was evaluated in order to determine that the extract only acts against tumor cells, for which purpose tumor cells were cultured for 72 hours under the above conditions in quantities of 200 µL of medium per well in 96-well, flat bottom, ultra-low adherence plates (Costar, Cambridge, MA, USA) with various doses of extract. Once elapsed that time, a direct count was carried out with hemocytometer and by trypan blue exclusion, which enters the cells having membrane damage, staining their nucleus in blue, indicating that they are not viable anymore; Bone marrow cell viability was evaluated after 5 days of culture.

The use of IC₅₀ values of the hybrid for each cell line, as described in Table 2, in tumor cell lines or in mouse bone marrow mononucleated cells, indicates the viability of normal cells, Table 4. L929 has a reduced viability (64%), followed by HeLa (87%), a reduction that also correlates to that observed with citarabine. In WEHI-3 and K562, the extract of *Sechium edule* hybrid named "H-387-07-GISeM" does not modify their viability, but Ara-c totally does so. That is, the extract of *Sechium edule* hybrid named "H-387-07-GISeM" only affects tumor cells and not normal bone marrow cells.

**Table 4. Percentage of viability of tumor cells treated with or without the IC₅₀ of the extract of H-387-07-GISeM with citarabine (Ara-C 5 µg/mL) or where applicable, imatinib. Values are presented as mean ± standard deviation.**

| **Cell line** | **% Viability** | | |
|---|---|---|---|
| | **0** | **H-387-GISeM** | **Ara-C** |
| **WEHI-3** | 97.4 ± 5.1 | 91.7 ± 10.5 | 0 ± 0 |
| **K-562** | 98.7 ± 6.7 | 92.8 ± 8.3 | 4 ± 2.3^{&} |
| **HeLa** | 97.6 ± 5.9 | 87.6 ± 9.6 | 83.2 ± 4.8 |
| **L929** | 95.6 ± 4.8 | 64.2 ± 8.2 | 82.4 ± 3.2 |
| **Bone Marrow normal** | 98.2 ± 4.6 | 100 ± 9.1 | 0 ± 0 |

| | | | |
|---|---|---|---|
| ^{&} The only cells treated with Imatinib (1 µM). | | | |

To confirm this, a treatment using female BALB/c mice aged 2-3 months was performed. Four groups of 5 mice each were formed. The extract (6.6 mg/kg in 1 mL PBS) of the hybrid H-387-07-GISeM or PBS was inoculated; a group was treated with citarabine as a cytotoxicity control. Four inoculations were performed, one each 48 hr; 24 hours after the last inoculation, cells were extracted and then were processed, to finally assess the mitotic index (1000 cells per slide were counted). To evaluate the toxic effect of long-term administration, groups of mice under the same conditions were injected with the extract each 48 hr for 6 months, weighed every week. Also their behaviour and survival 3 months further from the last inoculation were determined. The extract of the hybrid H-387-07-GISeM is not cytotoxic for normal bone marrow cells. To evaluate the cytotoxic effect in vivo, healthy mice were treated with the extract of the hybrid H-387-07-GISeM (6.6 mg/kg) each 48 hr for 7 days. The percentage of mitotic indexes on bone marrow leukocytes was quantified in these mice. It was found that the extract of *Sechium edule* hybrid named "H-387-07-GISeM" does not reduce the mitotic index in comparison to the control mouse, while Ara-c does, which indicates that the extract is not cytotoxic.

This same treatment regime was applied, but for a period of 25 weeks, and with 11-week monitoring after the completion of treatment. Mice were maintained alive and under conditions similar to that of untreated mice, which indicates that the doses used are not cytotoxic and do not cause damage under long-term intraperitoneal administration.

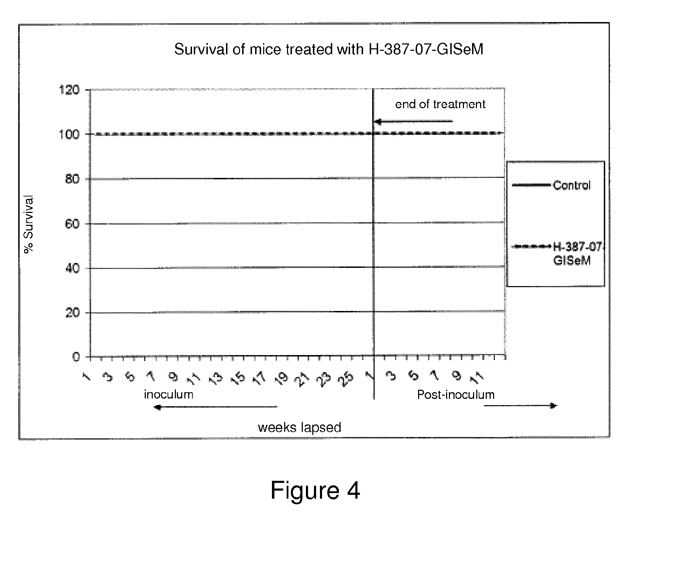

Figure 4 depicts the survival of mice treated with 6.6 mg/kg of the extract H-387-07-GISeM (CUCURBIN-387) each 48 hr, during 25 weeks and maintained for 12 weeks post inoculum.

## Claims

1. A crude extract of *Sechium edule* hybrid H-387-07-GISeM, wherein said extract has antineoplastic therapeutic effect.

2. The crude extract of *Sechium edule* hybrid H-387-07-GISeM according to claim 1, wherein it is chemically composed of terpenes, phenols, flavonoids, tannins, saponins and fatty acid esters.

3. The crude extract of *Sechium edule* hybrid H-387-07-GISeM according to claim 2, wherein it is chemically composed of terpenes in a proportion of about 29%, phenols in a proportion of about 21%, flavonoids in a proportion of about 14%, tannins in a proportion of about 8%, saponins in a proportion of about 14%, and fatty acid esters in a proportion of about 14%.

4. The crude extract of *Sechium edule* hybrid H-387-07-GISeM according to claim 1, wherein said extract is useful for the treatment of liquid and solid tumors that cause many types of cancer.

5. The crude extract of *Sechium edule* hybrid H-387-07-GISeM according to claim 1, wherein the administration of said extract does not have side effects on normal cells.

6. A method for obtaining the crude extract of *Sechium edule* hybrid H-387-07-GISeM according to claim 1, wherein said method comprises the following steps:
a) Cutting off fruits of the hybrid H-387-07-GISeM and cutting them into small pieces,
b) Drying said pieces and grinding them,
c) Extracting the dry material through a discontinuous extraction with an organic or aqueous solvent,
d) Evaporating the solvent,
e) Dissolving in ethanol,
f) Diluting to the volume with a phosphate buffer solution and homogenizing,
g) Centrifuging and collecting the supernatant,
h) Making dilutions.
i) Sterilizing with UV light; and,
j) Storing.

7. The method for obtaining a crude extract of the hybrid H-387-07-GISeM according to claim 6, wherein in the step a) the fruit includes epidermis, prickles and seed.

8. The method for obtaining a crude extract of the hybrid H-387-07-GISeM according to claim 6, wherein in the step c) said solvent can be methanol, ethanol, hexane or water.

9. The method for obtaining a crude extract of the hybrid H-387-07-GISeM according to claim 8, wherein preferably the solvent is ethanol.

10. The method for obtaining a crude extract of the hybrid H-387-07-GISeM according to claim 6, wherein in the step h) the dilutions are carried out using the supernatant.

11. Use of a crude extract of the hybrid H-387-07-GISeM according to any according to claims 1 to 5, for the preparation of a pharmaceutical composition with high antineoplastic effect.
